# NEUE EUROPÄISCHE PATENTSCHRIFT

(11) **EP 2 674 480 B2**
(45) Veröffentlichungstag und Bekanntmachung des Hinweises auf die Entscheidung über den Einspruch: **05.04.2023**
(45) Hinweis auf die Patenterteilung: 07.01.2015
(21) Anmeldenummer: 12172291.2
(22) Anmeldetag: 15.06.2012
(51) Int. Cl.: C12M 1/04

(54) **Anschlussvorrichtung für eine sterile Einweg-Fluidleitung eines Einwegbioreaktors und Verfahren zum Behandeln eines Fluidstroms**
Connection device for a sterile disposable fluid conduit of a disposable bioreactor and method for treating a fluid flow
Dispositif de raccordement pour la conduite de fluide stérile et à usage unique d'un bioréacteur jetable et procédé pour le traitement d'un flux de liquide

(43) Veröffentlichungstag der Anmeldung: 18.12.2013
(73) Patentinhaber: DASGIP Information and Process Technology GmbH, 52428 Jülich (DE)
(72) Erfinder: Arnold, Matthias, 52074 Aachen (DE); Köhn, Heinz-Gerhard, 22339 Hamburg (DE); Gülzow, Nico, 22337 Hamburg (DE); Eikelmann, Sven, 22299 Hamburg (DE)
(74) Vertreter: Eisenführ Speiser

(56) Entgegenhaltungen:
- EP-A1- 2 251 407
- WO-A1-2011/041508
- WO-A2-2008/088379
- DE-A1- 3 714 671
- DE-A1- 3 720 049
- DE-U1-202009 006 839
- DE-U1-202009 016 783
- DE-U1-202010 007 615
- GB-A- 2 465 282
- US-A- 5 443 985
- US-A1- 2003 079 787
- US-A1- 2010 301 060
- US-A1- 2012 003 733
- US-B1- 6 432 698

## Beschreibung

### Hintergrund der Erfindung

Die Erfindung gemäß der vorliegenden Ansprüche betrifft eine wiederverwendbare Temperiereinrichtung zur Temperierung einer sterilen Einweg-Fluidleitung eines Einwegbioreaktors, und eine biotechnologische Vorrichtung umfassend einen Einwegbioreaktor mit einer sterilen Einweg-Fluidleitung.

Bioreaktoren, die häufig auch als Fermenter bezeichnet werden, schließen einen Reaktionsraum ein, in dem biologische bzw. biotechnologische Prozesse im Labormaßstab durchgeführt werden können. Zu solchen Prozessen zählt beispielsweise die Kultivierung von Zellen, Mikroorganismen oder kleinen Pflanzen unter definierten, vorzugsweise optimierten, kontrollierten und reproduzierbaren Bedingungen. Bioreaktoren verfügen dazu meist über mehrere Anschlüsse, über die Primär- und Sekundärstoffe sowie verschiedene Instrumente, wie beispielsweise Sensoren, in den Reaktionsraum eingebracht werden können oder über die beispielsweise Fluidleitungen angeschlossen werden können. Durch solche Fluidleitungen, insbesondere Gasleitungen, können Fluide, insbesondere Gase, dem Reaktionsraum zu- oder abgeführt werden. Je nach Fluidstromrichtung können Fluidleitungen als Zu- oder Ableitungen bezeichnet werden. Gasleitungen beispielweise können je nach Gasstromrichtung als Zugas-bzw. Begasungsleitungen oder Abgasleitungen bezeichnet werden.

Sowohl für den Anwendungsbereich der Zellkultivierung als auch für den mikrobiologischen Anwendungsbereich ist die Verwendung von Bioreaktoren in, vorzugsweise parallelen, Bioreaktorsystemen bevorzugt. Parallele Bioreaktorsysteme sind beispielsweise in der DE 10 2011 054 363.5 oder der DE 10 2011 054365.1 beschrieben. In einem solchen Bioreaktorsystem können mehrere Bioreaktoren parallel betrieben und mit hoher Genauigkeit kontrolliert werden. Dabei können auch bei kleinen Arbeitsvolumina in den einzelnen Bioreaktoren Experimente mit hohem Durchsatz durchgeführt werden, die gut reproduzierbar und skalierbar sind.

Im Anwendungsbereich der Zellkultur werden solche parallelen Bioreaktorsysteme beispielsweise für auf statistischen Planungsmethoden (Design of Experiments DoE) basierenden Versuchsreihen zur Prozessoptimierung, die Prozessentwicklung sowie die Forschung und Entwicklung eingesetzt, beispielsweise um verschiedene Zelllinien, wie Chinese Hamster Ovary (CHO)-, Hybridoma- oder NSO-Zelllinien, zu kultivieren. Unter dem Begriff "Zellkultur" wird im Rahmen des vorliegenden Textes insbesondere die Kultivierung tierischer oder pflanzlicher Zellen in einem Nährmedium außerhalb des Organismus verstanden.

Im Anwendungsbereich der Mikrobiologie werden parallele Bioreaktorsysteme ebenfalls für auf statistischen Planungsmethoden (Design of Experiments DoE) basierenden Versuchsreihen zur Prozessoptimierung, die Prozessentwicklung sowie die Forschung und Entwicklung eingesetzt, beispielsweise um verschiedene Mikroorganismen, insbesondere Bakterien oder Pilze, z.B. Hefen, zu kultivieren.

Bioreaktoren im Laboreinsatz sind oft aus Glas und/oder Metall, insbesondere rostfreiem Stahl, ausgebildet, da die Bioreaktoren zwischen verschiedenen Anwendungen sterilisiert werden müssen, was vorzugsweise durch eine Heißdampfsterilisation In einem Autoklaven erfolgt. Die Sterilisierung und Reinigung wiederverwendbarer Bioreaktoren ist aufwändig: Der Sterilisations- und Reinigungsprozess kann einer Validierung unterliegen und seine Durchführung ist für jeden einzelnen Bioreaktor genau zu dokumentieren. Rückstände in einem nicht vollständig sterilisierten Bioreaktor können die Ergebnisse eines nachfolgenden Prozesses verfälschen oder unbrauchbar machen und einen nachfolgenden Prozessablauf stören. Des Weiteren können einzelne Bestandteile bzw. Materiaiien der Bioreaktoren durch den Sterilisationsprozess beansprucht und z.T. beschädigt werden.

Eine Alternative zu wiederverwendbaren Bioreaktoren stellen Einwegbioreaktoren dar, die für die Durchführung lediglich eines biologischen bzw. biotechnologischen Prozesses verwendet und anschließend entsorgt werden. Durch die Bereitstellung eines neuen, vorzugsweise im Herstellungsprozess sterilisierten, Einwegbioreaktors für jeden Prozess kann die Gefahr einer (Kreuz-)Kontamination reduziert werden und gleichzeitig entfällt der Aufwand der Durchführung und Dokumentation einer einwandfreien Reinigung und Sterilisierung eines zuvor genutzten Bioreaktors. Einwegbioreaktoren sind oft als flexible Behälter ausgebildet, beispielsweise als Beutel oder als Behälter mit zumindest abschnittsweise flexiblen Wänden. Beispiele für solche Bioreaktoren sind in US 2011/0003374 A1, US2011/0058447A1, DE 20 2007 005 868U1, US 2011/0058448A1, US2011/0207218A1, WO 2008/088379A2, US 2012/0003733 A1, WO2011/079180A1, US2007/0253288A1, US 2009/0275121A1 und US 2010/0028990A1 beschrieben. Formstabile Einwegbioreaktoren sind beispielsweise aus der EP 2 251 407 A1 und der US 2009/0311776 A1 bekannt.

Ein steriler Einwegbioreaktor umfasst in der Regel eine daran angeschlossene, ebenfalls sterilisierte und für einen einmaligen Gebrauch vorgesehene Fluidleitung, insbesondere eine oder mehrere Gasleitungen. Eine solche Einweg-Fluidleitung kann als formstabiles Rohr oder als flexibler Schlauch ausgebildet sein. An einer Einweg-Fluidleitung, vorzugsweise an dem nicht am Bioreaktor angeschlossenen Ende der Fluidleitung, ist meist ein Sterilfilter vorgesehen, der ebenfalls meist sterilisiert und für einen einmaligen Gebrauch vorgesehen ist. Insbesondere in einer Gasleitung dient ein Sterilfilter dazu, in einen Bioreaktor durch eine Begasungsleitung einströmendes Gas oder aus einer Abgasleitung aus einem Bioreaktor austretendes Gas zu filtern.

In der Anwendung von Bioreaktoren in biologischen bzw. biotechnologischen Prozessen, beispielsweise zur Kultivierung verschiedener Mikroorganismen, insbesondere Bakterien oder Pilze, z.B. Hefen, oder zur Kultivierung verschiedener Zelllinien, wie Chinese Hamster Ovary (CHO)-, Hybridoma- oder NSO-Zelllinien ist es wichtig, dass die Sterilität des Einwegbioreaktors und seiner Komponenten nicht beeinträchtigt wird. Unter dem Begriff "Zellkultur" wird im Rahmen des vorliegenden Textes insbesondere die Kultivierung tierischer oder pflanzlicher Zellen in einem Nährmedium außerhalb des Organismus verstanden.

Solche biologischen bzw. biotechnologischen Prozesse werden in der Regel unter sterilen Bedingungen in wässrigen Medien, die auch als Kulturbrühe bezeichnet werden können, in einem Reaktionsraum eines Bioreaktors durchgeführt. Zumindest während eines bestimmten Zeitraums der Kultivierung werden dem System zur Aufrechterhaltung der biologischen Vorgänge gasförmige Fluide zugeführt, beispielsweise Gemische aus Stickstoff, Sauerstoff, Kohlendioxid, etc., und/oder gasförmige Fluide, wie beispielsweise Methan, Kohlendioxid, etc., abgeführt, welche auch durch biologische Vorgänge erzeugte weitere Bestandteile enthalten können. Die sterilen Bedingungen im Reaktionsraum eines Bioreaktors und somit auch des biologischen Systems werden sowohl bei der Zu- als auch bei der Abfuhr von Fluiden in der Regel durch in den Fluid-Strom geschaltete Sterilfilter aufrecht erhalten. Typischerweise ist das abgeführte gasförmige Fluid weitgehend mit Wasserdampf gesättigt. Meist liegt die Temperatur der wässrigen Kulturbrühe über der Temperatur der Systemumgebung, beispielsweise einem Labor. In diesen Fällen wird das abgeführte gasförmige Fluid beim Austritt aus dem Reaktionsraum des Bioreaktors in den Abgasschlauch zumindest geringfügig abgekühlt. Hierdurch kondensiert ein Teil des im Gas enthaltenen Wasserdampfs. Eine Rückführung des Kondensats in den Reaktionsraum ist wünschenswert, da ansonsten eine Aufkonzentration von Medienbestandteile in der Kulturbrühe über den zeitlichen Verlauf des Prozesses erfolgen kann, was zum Beispiel zu einem unerwünschten Anstieg der Osmolalität führen kann. Ein weiterer Nachteil ist, dass der Sterilfilter durch gebildetes Kondensat blockiert und dadurch das gasförmige Fluid nicht mehr abgeführt werden kann, der Abgasstrom somit blockiert ist. Dies kann dazu führen, dass auch die Gaszufuhr in den Reaktionsraum blockiert wird und damit der Kultivierungsprozess gestört oder beendet wird.

Für wiederverwendbare Bioreaktoren werden daher Kühlvorrichtungen in der Abgasführung eingesetzt, was beispielsweise in der US 2012/0103579 A1, US 2010/0170400 A1 und US 2011/0076759 A1 gezeigt ist. Eine Abgaskühlung für Einwegbioreaktoren wird beispielsweise in der WO 2011/041508 A1 vorgeschlagen.

In Einwegbioreaktoren hat sich der Einsatz von Systemen zur Abgaskühlung in der Praxis jedoch insbesondere aufgrund der Anforderungen an die Beibehaltung der Sterilität bisher nicht durchgesetzt, was zu den genannten Nachteilen des Wasserentzugs und einer Filter-Verblockung führen kann. Teilweise wird in existierenden Lösungen versucht, eine Verblockung eines Sterilfilters zu verhindern, indem durch die Beheizung des Abgases im Bereich des Abgasschlauchs die relative Feuchte des Abgasstroms so weit abgesenkt wird, dass dieser ohne Kondensation, den Sterilfilter passieren kann. Dabei bleibt der Nachteil bestehen, dass kein Kondensat aus dem Abgas zurückgewonnen und der Kulturlösung zurückgeführt werden kann. Ferner kann es auch bei diesen Lösungen weiterhin zu einem Verblocken des Sterilfilters kommen. Daher werden zur Erhöhung der Prozesssicherheit anstelle von günstigen, jedoch zur Verblockung neigenden Membranfiltern oft aufwändige Einweg-Sterilfilter, wie beispielsweise Tiefenfilter oder Kapselfilter eingesetzt, was jedoch zu höheren Kosten der Einwegbioreaktoren führt. Ferner wird versucht, durch eine Beheizung des Sterilfilters selbst eine Verblockung desselben durch Kondensat zu verhindern, indem die Temperatur im Sterilfilter heraufgesetzt wird, um Kondensatbildung zu verringern oder zu vermeiden. Dies hat ebenfalls unter anderem den Nachteil, dass kein Kondensat aus dem Abgas zurückgewonnen und der Kulturlösung zurückgeführt werden kann, und auch mit diesem Lösungsansatz ein Verblocken des Sterilfilters nicht zuverlässig genug verhindert werden kann.

Eine biotechnologische Vorrichtung mit einer Temperiereinrichtung ist auch aus der DE 10 2011 054 364.3 bekannt. Während die darin beschriebene biotechnologische Vorrichtung sowie die ebenfalls beschriebene Temperiereinrichtung insbesondere bei autoklavierbaren Bioreaktoren Vorteile bietet, ist es jedoch wünschenswert, die AbgasTemperierung für sterile Einwegbioreaktoren weiter zu vereinfachen und zu verbessern, insbesondere hinsichtlich der Prozesssicherheit und Energieeffizienz. Insbesondere die Sterilität eines Einwegbioreaktors und eines daran angeordneten sterilen Einweg-Abgasschlauchs sollte durch das Vorsehen einer Abgastemperierung nicht negativ beeinflusst werden.

DE202009016783U lehrt ein komplexes Abgassystem eines Bioreaktors mit der Aufteilung eines Gasabführungskanals in eine Vielzahl von Unterkanälen. Der Gasabführungskanal zum Austrag von Abgas aus einem Bioreaktorbehälter ist mit einem davor liegenden Wärmetauscher ausgestattet, wobei vor der Öffnung des wenigstens einen Gasabführungskanals zur Umgebung hin ein hydrophober Sterilfilter angeordnet ist.

Ein weiterer Nachteil von bekannten Lösungen besteht darin, dass durch die Zufuhr von, beispielsweise gekühlten, Fluiden, insbesondere Gasen, in den Reaktionsraum die Temperatur im Reaktionsraum beeinflusst bzw. verändert werden kann, was sich insbesondere bei temperaturempfindlichen Prozessen negativ auswirken kann und beispielsweise zu einem erhöhten Temperieraufwand der Kulturbrühe führen kann. Auch die Temperaturregelung kann durch die Zufuhr von, beispielsweise gekühlten, Fluiden, insbesondere Gasen, in den Reaktionsraum negativ beeinflusst werden, insbesondere durch kalte Gasimpulse. Ferner können auch in Zugas- bzw. Begasungsleitungen Filter vorgesehen sein, die durch Kondensatbildung blockiert werden können. Insbesondere bei der Zufuhr von feuchtem Gas oder Gasgemischen, beispielsweise bei der Zufuhr des Abgases eines vorgeschalteten Prozesses als Zugas in einen nachgeschalteten Bioreaktor, kann die Ausfilterung von Gasbestandteilen mittels eines Filters gewünscht sein, wofür ebenfalls eine Verblockung des Filters nachteilig ist.

Es ist daher eine Aufgabe der vorliegenden Erfindung, die Fluidtemperierung von zu- oder abströmenden Fluiden, insbesondere Gasen, in einer sterilen Einweg-Fluidleitung, insbesondere von Abgas in einer sterilen Einweg-Gasleitung, eines Einwegbioreaktors zu verbessern, um einen oder mehrere der genannten Nachteile zu vermeiden oder zu verringern.

### Zusammenfassung der Erfindung

Die Erfindung betrifft eine wiederverwendbare Temperiereinrichtung nach Anspruch 1 und eine biotechnologische Vorrichtung nach Anspruch 5.

Beschrieben ist eine wiederverwendbare Anschlussvorrichtung für eine sterile Einweg-Gasleitung eines Einwegbioreaktors, umfassend eine Aufnahme, in der ein Abschnitt einer Gasleitung lösbar angeordnet werden kann, und ein Kopplungselement mit einer Koppelfläche zur Verbindung der Anschlussvorrichtung mit einer Temperiereinrichtung, wobei die Aufnahme eine Kontaktfläche aufweist, die derart angeordnet und ausgebildet ist, dass die Kontaktfläche an einem Abschnitt einer in der Aufnahme angeordneten Gasleitung anliegt, und wobei ein Abschnitt einer Gasleitung in einer im Wesentlichen orthogonal zur einer Längsachse der Anschlussvorrichtung verlaufenden Richtung in die Aufnahme eingeführt werden kann.

Im hier vorliegenden Text werden die Ausführungsformen und Vorteile der Erfindung insbesondere für die Gasführung, und ferner insbesondere für die Abgasführung, beschrieben. Die Ausführungsformen und Vorteile können jedoch ebenso für Gasleitungen mit anderer Strömungsrichtung, insbesondere auch Zugas- bzw. Begasungsleitungen, realisiert werden. Ebenso können die hier genannten Ausführungsformen und Vorteile für Fluidleitungen im Allgemeinen realisiert werden.

Ebenso werden die Ausführungsformen und Vorteile der Erfindung insbesondere für die Abgastemperierung eines Abgasstroms mittels einer Temperiereinrichtung beschrieben. Auch hier können die jeweiligen Ausführungsformen und Vorteile auch für die Temperierung von Fluiden gleich welcher Strömungsrichtung realisiert werden.

Die Erfindung beruht auf der Erkenntnis, dass einerseits eine Beeinträchtigung der Sterilität eines Einwegbioreaktors und einer daran angeschlossenen Gasleitung vermieden und andererseits eine kostengünstige Lösung zur Gastemperierung dadurch erzielt werden kann, dass ein bekannter Einwegbioreaktor mit einer üblicherweise daran angeschlossenen sterilen Einweg-Gasleitung unverändert zum Einsatz kommen kann, indem daran eine wiederverwendbare Anschlussvorrichtung angeordnet wird. Auf diese Weise können herkömmliche Einwegbioreaktoren verwendet werden, ohne durch spezielle Vorrichtungen deren Herstellungskosten zu erhöhen. Gleichzeitig werden durch die Wiederverwendbarkeit der Anschlussvorrichtung die Kosten der Gastemperierung niedrig gehalten.

Die Anschlussvorrichtung ist mit einer Aufnahme ausgebildet, in der ein Abschnitt der Gasleitung, insbesondere ein Abschnitt eines Abgasschlauchs, lösbar angeordnet werden kann. Eine Relativbewegung zwischen der Gasleitung, insbesondere dem Abgasschlauch, und der Anschlussvorrichtung erfolgt im Wesentlichen orthogonal zu einer Längsachse der Anschlussvorrichtung, d.h. dass die Gasleitung in einer im Wesentlichen radialen Richtung (bezogen auf eine Leitungsachse bei gerader Leitungsanordnung) in die Aufnahme eingeführt werden kann. Die Längsachse der Anschlussvorrichtung wird hier verstanden als Achse in der Richtung, in der die Anschlussvorrichtung ihre größte Erstreckung hat. Vorzugsweise ist die Längsachse der Anschlussvorrichtung identisch mit oder parallel zu einer Leitungsachse der Gasleitung bei gerader Leitungsanordnung.

Auf diese Weise wird ein einfaches Verbinden und Lösen von Gasleitung und Anschluss vorrichtung realisiert. Das Anbringen und Lösen der Anschlussvorrichtung mit Aufnahme an der Gasleitung erfolgt dabei vorzugsweise nach und nach in einer Richtung im Wesentlichen orthogonal zur Erstreckung der Aufnahme. Dies ist insbesondere vorteilhaft, da bei einer solchen Anordnung in besonders einfacher Weise sichergestellt ist, dass keine Demontage der Gasleitung vom Einwegbioreaktor oder des Sterilfilters von der Gasleitung erforderlich ist und somit die Sterilität des Systems gewährleistet bleibt. Nach der Bereitstellung eines sterilen Einwegbioreaktors kann somit für die Prozessdurchführung an dem sterilen Einweg-Abgasschlauch die erfindungsgemäße Anschlussvorrichtung angeordnet werden, indem ein Abschnitt der Gasleitung in die Aufnahme der Anschlussvorrichtung eingeführt wird bzw. die Anschlussvorrichtung mit Aufnahme an der Gasleitung angebracht wird. Nach Beendigung der Prozessdurchführung und vor der Entsorgung des Einwegbioreaktors kann die Anschlussvorrichtung wieder von der Gasleitung abgenommen werden und steht für eine erneute Verwendung zur Verfügung. Die Sterilität des Einweg-Gasleitung oder des Einwegbioreaktors wird durch das lösbare Anbringen der Anschlussvorrichtung nicht beeinträchtigt.

Eine Fluidleitung ist eine als flexibler Schlauch oder als formstabiles Rohr ausgebildete Gasleitung.

Bei einer Ausbildung als flexibler Schlauch ist eine formstabile Ausbildung der Anschlussvorrichtung besonders bevorzugt. Bei einer Ausbildung der Gasleitung als Rohr ist eine Ausbildung der Anschlussvorrichtung, insbesondere der Aufnahme und der Kontaktfläche, aus einem, vorzugsweise elastisch, verformbaren Material bevorzugt. Mit diesen Kombinationen kann in vorteilhafter Weise ein einfaches Verbinden und Lösen von Gasleitung und Anschlussvorrichtung realisiert werden, da bei den genannten Kombinationen Gasleitung und/oder Anschlussvorrichtung eine gewisse Verformbarkeit erlauben, die eine leichte Herstellung und Lösung beispielsweise eines Presssitzes oder einer Klemmung erlauben.

Die Anschlussvorrichtung weist ferner ein Kopplungselement auf, mit dem die Anschlussvorrichtung an eine Temperiereinrichtung angeschlossen werden kann. Mit einer solchen Temperiereinrichtung kann eine Gasleitung, und darüber auch ein in der Gasleitung strömender Gasstrom, temperiert werden. Im Gegensatz zu der lösbaren Anordnung der Gasleitung in der Aufnahme kann die Verbindung zwischen dem Kopplungselement und einer Temperiereinrichtung lösbar oder nicht lösbar ausgebildet sein. Vorzugsweise ist die Verbindung zwischen dem Kopplungselement und einer Temperiereinrichtung eine thermisch koppelnde Verbindung, sodass eine gute Wärmeleitfähigkeit zwischen der Anschlussvorrichtung und der Temperiereinrichtung gewährleistet ist. Das Koppelelement weist eine, vorzugsweise ebene, Kopplungsfläche auf, die mit einer Temperierfläche der Temperiereinrichtung in flächigen Kontakt gebracht werden kann.

Die Aufnahme der Anschlussvorrichtung weist eine Kontaktfläche auf, die an der Außenfläche einer in der Aufnahme angeordneten Gasleitung anliegt. Im Querschnitt der Aufnahme bildet die Kontaktfläche vorzugsweise einen im Wesentlichen kreisbogenförmigen Kontaktabschnitt. Die Kontaktfläche ergibt sich aus dem vorzugsweise kreisbogenförmigen Kontaktabschnitt im Querschnitt und einer Längserstreckung der Aufnahme in Richtung einer Längsachse. Der Radius des Kontaktabschnitts der Aufnahme im Querschnitt ist vorzugsweise an den Radius einer Gasleitung angepasst ist, um den Kontakt zwischen der Kontaktfläche mit einer entsprechenden Fläche an der Außenoberfläche der Gasleitung zu ermöglichen. Die Kontaktfläche liegt vorzugsweise vollflächig an einem entsprechenden Flächenabschnitt der Außenoberfläche der Gasleitung an. Ein solcher vollflächiger Kontakt ermöglicht eine gute Wärmeübertragung zwischen der Anschlussvorrichtung und der Gasleitung. Dies ist vorteilhaft, um eine Temperierleistung, insbesondere eine Kühlleistung, einer an dem Kopplungselement angeschlossenen Temperiereinrichtung wirksam auf die Gasleitung und einen darin strömenden Gasstrom übertragen zu können.

Die Anschlussvorrichtung hat ferner den Vorteil, den in der Aufnahme angeordneten Abschnitt der Gasleitung zu stabilisieren und damit ein Abknicken der Gasleitung, insbesondere wenn diese als flexibler Schlauch ausgebildet ist, zu verhindern. Diese Stabilisierung, insbesondere wenn sie in einer entsprechenden Richtung erfolgt, kann auch einen gravitationsbedingten Rückfluss von in der Gasleitung gebildetem Kondensat in den Einwegbioreaktor fördern.

Die Anschlussvorrichtung hat darüber hinaus den Vorteil, dass sie den Einsatzbereich von Einwegbioreaktoren erweitert auf sterile Applikationen, bei denen eine effiziente Rückgewinnung von Flüssigkeiten aus dem Abgas obligatorisch ist.

Gemäß Anspruch 1 weist die Aufnahmeeinen ersten und einen zweiten Teil auf, wobei die beiden Teile ausgebildet sind, in einer im Wesentlichen orthogonal zu einer Längsachse der Anschlussvorrichtung verlaufenden Richtung zusammengefügt zu werden und dabei einen Abschnitt einer Fluidleitung, insbesondere einer Gasleitung, in der Aufnahme aufzunehmen. Auch in dieser Ausführungsform wird die Anordnung der Gasleitung in der Aufnahme durch eine bezogen auf eine Leitungsachse der Gasleitung im Wesentlichen radialen Richtung vollzogen, wodurch ebenfalls in vorteilhafter Weise die Beibehaltung der Sterilität des Systems erzielt wird, insbesondere durch die Vermeidung einer Trennung von Gasleitung und Sterilfilter sowie Gasleitung und Einwegbioreaktor.

In einer bevorzugten Ausführungsform ist die Aufnahme derart ausgebildet, dass ein Abschnitt einer Fluidleitung, insbesondere ein Abschnitt einer Gasleitung, darin klemmend und/oder durch Formschluss gehalten werden kann. Durch diese Verbindungsmöglichkeiten zwischen Aufnahme und Fluid- bzw. Gasleitung kann eine besonders gute thermische Ankopplung erzielt und damit eine besonders gute Wärmeübertragung erreicht werden.

Bevorzugt ist insbesondere, dass ein flexibler Schlauch beim Einbringen in die Aufnahme elastisch verformt wird und durch eine Rückverformung in der Aufnahme dann ein entsprechender Flächenabschnitt der Außenoberfläche des Schlauchs mit der Kontaktfläche der Aufnahme in Kontakt kommt.

Eine alternative bevorzugte Ausführungsform sieht vor, dass sich beim Anbringen der Aufnahme der Anschlussvorrichtung an ein formstabiles Rohr die Anschlussvorrichtung mit der Aufnahme elastisch verformt und sich die Kontaktfläche der Aufnahme um einen entsprechenden Flächenabschnitt der Außenoberfläche des Rohrs legt.

Insbesondere ist es bevorzugt, dass die Anschlussvorrichtung aus einem Material mit einer hohen Wärmeleitfähigkeit ausgebildet ist oder ein solches Material aufweist. Bevorzugt ist eine Wärmeleitfähigkeit λ von über 10 W/(m*K). Besonders bevorzugt ist eine Wärmeleitfähigkeit λ von über 15 W/(m*K), insbesondere von über 25 W/(m*K), über 50 W/(m*K), über 75 W/(m*K), über 100 W/(m*K), über 150 W/(m*K) oder über 200 W/(m*K). Besonders bevorzugt ist es, dass die Anschlussvorrichtung aus Aluminium ausgebildet ist oder Aluminium aufweist. Andere bevorzugte Materialien für die Anschlussvorrichtung sind Metalle, wie beispielsweise Edelstahl, oder andere hoch thermisch leitfähige Stoffe. Beispielsweise können auch Kompositwerkstoffe mit einer Kunststoffmatrix und einem darin eingebetteten, hoch thermisch leitfähigen Material zum Einsatz kommen, insbesondere wenn eine elastische Verformbarkeit der Anschlussvorrichtung bevorzugt ist.

Ferner ist es bevorzugt, dass die Anschlussvorrichtung eine Längserstreckung aufweist, die kürzer ist als eine Erstreckung einer Fluidleitung zwischen einer Verbindung der Fluidleitung mit einem Einwegbioreaktor und einem an der Fluidleitung angeordneten Sterilfilter. Insbesondere ist es bevorzugt, dass die Anschlussvorrichtung eine Längserstreckung aufweist, die kürzer ist als eine Erstreckung einer Gasleitung zwischen einer Verbindung der Gasleitung mit einem Einwegbioreaktor und einem an der Gasleitung angeordneten Sterilfilter.

Auf diese Weise kann sichergestellt werden, dass die Anschlussvorrichtung nicht länger ist als der für den Anschluss zur Verfügung stehende Abschnitt der Gasleitung. Ferner ist jedoch bevorzugt, dass die Längserstreckung der Anschlussvorrichtung einen Großteil der Länge der Gasleitung zwischen ihrer Verbindung mit dem Einwegbioreaktor und einem Sterilfilter ausmacht, da auf diese Weise die für eine Wärmeübertragung zur Verfügung stehende Kontaktfläche vergrößert werden kann.

Vorzugsweise weisen die Aufnahme und die Kontaktfläche die gleiche Längserstreckung auf, d.h. der, vorzugsweise im Wesentlichen kreisbogenförmige, Kontaktabschnitt im Querschnitt der Aufnahme ist vorzugsweise über die gesamte Länge der Aufnahme ausgebildet.

Ferner ist bevorzugt, dass die Anschlussvorrichtung einen Verbindungsabschnitt aufweist, mit dem die Anschlussvorrichtung lösbar an einem Anschlusselement eines Einwegbioreaktors befestigbar ist. Diese Befestigung ist vorzugsweise eine lösbare Steckverbindung, wobei der Verbindungsabschnitt der Anschlussvorrichtung vorzugsweise in ein entsprechendes Anschlusselement des Einwegbioreaktors einsteckbar ist. Das Anschlusselement kann beispielsweise auf einer Kopfplatte eines Einwegbioreaktors angeordnet sein.

Durch die Verbindung der Anschlussvorrichtung über einen Verbindungsabschnitt mit einem Anschlusselement des Einwegbioreaktors kann die Anschlussvorrichtung mit der darin lösbar eingelegten Gasleitung, insbesondere eines flexiblen Schlauchs, stabilisiert werden, vorzugsweise in einer Position, die einen Rücklauf des in der Gasleitung gebildeten Kondensats durch Gravitation in den Reaktionsraum des Einwegbioreaktors erleichtert.

Die Anschlussvorrichtung weist ferner vorzugsweise ein thermisches Isolierelement auf, das mit der Anschlussvorrichtung lösbar verbunden ist. Dieses thermische Isolierelement dient dazu, die Gasleitung und ggf. teilweise auch die diese Gasleitung umgebende Anschlussvorrichtung zu isolieren. Eine thermische Isolierung des Gasstromes dient insbesondere dazu, Kondensat an der Außenseite der Gasleitung und/oder der Anschlussvorrichtung zu verhindern oder zumindest zu verringern.

Das thermische Isolierelement kann vorzugsweise durch Formschluss und/oder Klemmung mit einem Abschnitt der Gasleitung und/oder der Anschlussvorrichtung verbunden werden. Das thermische Isolierelement ist vorzugsweise lösbar mit der Gasleitung und/oder der Anschlussvorrichtung verbunden und wiederverwendbar ausgebildet. Die Verbindung kann beispielsweise dadurch hergestellt werden, dass das thermische Isolierelement, zumindest in Grenzen, elastisch verformbar ist und durch eine entsprechende Verformung, beispielsweise eine Aufweitung, an einem Abschnitt der Gasleitung und/oder der Anschlussvorrichtung angeordnet werden kann und nach der Rückverformung eine Klemmung und/oder ein Formschluss realisiert ist.

Das thermische Isolierelement kann aus zwei Teilen ausgebildet sein, die vorzugsweise in einer im Wesentlichen orthogonal zu einer Längsachse des thermischen Isolierelements, die vorzugsweise mit einer Längsachse der Anschlussvorrichtung übereinstimmt oder parallel zu dieser verläuft, zusammengefügt werden und dabei einen Abschnitt der Gasleitung und/oder einen Teil der Anschlussvorrichtung umschließen.

Das thermische Isolierelement kann vorzugsweise schlecht wärmeleitende Materialien, vorzugsweise mit eingeschlossenen isolierenden Hohlräumen, wie beispielsweise geschäumte Polymere, aufweisen oder daraus bestehen.

Gemäß der Erfindung wird die Aufgabe gelöst durch eine wiederverwendbare Temperiereinrichtung gemäß Anspruch 1.

Bevorzugt ist insbesondere, dass der Temperieraktor an dem Kopplungselement der Anschlussvorrichtung, vorzugsweise thermisch koppelnd, montierbar ist. Besonders bevorzugt ist ferner, dass der Temperieraktor an dem Kopplungselement der Anschlussvorrichtung lösbar montierbar ist. Eine solche lösbare Verbindung kann beispielsweise eine form- und/oder kraftschlüssige Verbindung sein und kann beispielsweise als Steckverbindung, Rastverbindung, Verschraubung oder eine Verbindung durch Aufschieben ausgebildet sein. Alternativ kann der Temperieraktor derart mit dem Kopplungselement verbunden sein, dass eine Demontage durch einen Benutzer nicht möglich ist. Dies kann als eine stoffschlüssige Verbindung zwischen Temperieraktor und Anschlussvorrichtung realisiert sein oder durch eine andere, grundsätzlich lösbare Verbindung, die für einen Benutzer im bestimmungsgemäßen Gebrauch jedoch nicht zugänglich bzw. nicht lösbar ist.

In einer bevorzugten Ausführungsform ist die Temperiereinrichtung gekennzeichnet durch mindestens ein Wärmeübertragungselement zur Temperierung, insbesondere Erwärmung, eines Sterilfilters eines Einwegbioreaktors, wobei das mindestens eine Wärmeübertragungselement mit dem Temperieraktor thermisch koppelnd verbunden ist. Besonders bevorzugt ist, dass Abwärme des Temperieraktors zur Erwärmung des mindestens einen Wärmeübertragungselements verwendet wird.

Dies hat den Vorteil, dass die bei der Abgaskühlung zur Kondensatrückgewinnung entstehende Abwärme nicht einfach nur abgeführt, sondern zur Beheizung des Sterilfilters genutzt wird. Durch diese Kombination kann die Prozesssicherheit weiter erhöht werden, da durch die Abgaskühlung nicht nur Kondensat gebildet und damit der Flüssigkeitsgehalt des Abgases reduziert wird, sondern durch die gleichzeitige Erwärmung des Sterilfilters verhindert wird, dass eventuell im Abgas verbliebener Wasserdampf zu einem Verblocken des Sterilfilters führt. Gleichzeitig wird hiermit eine energieeffiziente Lösung geschaffen, bei der die bei der Kühlung entstehende Abwärme für die Erwärmung des Wärmeübertragungselements genutzt wird. Die thermische Kopplung des Wärmeübertragungselements und des Temperieraktors, vorzugsweise der warmen Seite des Temperieraktors, erfolgt vorzugsweise über einen wärmeleitenden Wärmeübertragungskörper, der auch als Kühlkörper bezeichnet werden kann, da seine primäre Funktion ist, die Abwärme vom Temperieraktor abzuführen. Es kann sich dabei um ein durchgehendes Bauteil handeln, das den Temperieraktor mit dem Wärmeübertragungselement verbindet.

In einer weiteren bevorzugten Ausführungsform ist die Temperiereinrichtung gekennzeichnet durch ein zweites Wärmeübertragungselement zur Temperierung, insbesondere Erwärmung, eines Sterilfilters eines Einwegbioreaktors, wobei das zweite Wärmeübertragungselement mit dem Temperieraktor thermisch koppelnd verbunden ist, wobei vorzugsweise das erste und zweite Wärmeübertragungselement derart angeordnet und ausgebildet sind, dass sie einen Sterilfilter zumindest teilweise umschließen können.

Besonders bevorzugt ist, dass Abwärme des Temperieraktors zur Erwärmung des zweiten Wärmeübertragungselements verwendet wird,

Diese Ausgestaltung erhöht sowohl die Prozesssicherheit als auch die Energieeffizienz weiter. Die Anordnung von zwei Wärmeübertragungselementen vorzugsweise auf zwei unterschiedlichen Seiten des Sterilfilters führt zu einer verbesserten, gleichmäßigeren Erwärmung des Sterilfilters.

Das erste und/oder zweite Wärmeübertragungselement können vorzugsweise plattenförmig, gegebenenfalls mit einem Schlitz zur Durchführung einer Gasleitung, ausgebildet sein. Ferner weisen das erste und/oder zweite Wärmeübertragungselement vorzugsweise auf ihrer jeweiligen einem Sterilfilter zugewandten Seite eine Vertiefung zur Aufnahme eines Teilbereichs des Sterilfilters auf, wobei die Vertiefung vorzugsweise im Wesentlichen als Negativform des aufzunehmenden Teilbereichs des Sterilfilters ausgebildet ist. Dies hat den Vorteil, dass ein verbesserter Formschluss zwischen dem ersten und/oder zweiten Wärmeübertragungselement und dem Sterilfilter erreicht wird und damit eine verbesserte Wärmeübertragung erzielt werden kann.

Ferner sind das erste und/oder zweite Wärmeübertragungselement vorzugsweise um eine horizontale Achse schwenkbar gelagert, um das Umschließen und Freigeben des Sterilfilters zu erleichtern. Diese schwenkbare Lagerung des ersten und/oder zweiten Wärmeübertragungselements erfolgt vorzugsweise an einem die Wärmeübertragungselemente mit dem Temperieraktor verbindenden Wärmeübertragungskörper.

Besonders bevorzugt ist es, dass die Temperiereinrichtung als temperierfluidfreie Abgastemperiereinrichtung, wie in der DE 10 2011 054 364.3 beschrieben, ausgebildet ist. Insbesondere ist es bevorzugt, einzelne oder mehrere der in der DE 10 2011 054 364.3 beschriebenen Ausführungsformen der Abgastemperiereinrichtung mit einzelnen oder mehreren zuvor beschriebenen Ausführungsformen der Anschlussvorrichtung zu kombinieren.

Gemäß einem weiteren Aspekt der Erfindung wird die Aufgabe gelöst durch eine biotechnologische Vorrichtung gemäß Anspruch 5.

Besonders bevorzugt ist es, dass die biotechnologische Vorrichtung als biotechnologische Vorrichtung, wie in der DE 10 2011 054 364.3 beschrieben, ausgebildet ist. Insbesondere ist es bevorzugt, einzelne oder mehrere der in der DE 10 2011 054 364.3 beschriebenen Ausführungsformen der biotechnologische Vorrichtung mit einzelnen oder mehreren zuvor beschriebenen Ausführungsformen der Anschlussvorrichtung bzw. der Temperiereinrichtung zu kombinieren.

Ferner ist es bevorzugt, dass der Einwegbioreaktor als Einwegbioreaktor, wie er in der parallelen Anmeldung der Anmelderin vom selben Tage mit dem Titel "Einwegbioreaktor und Kopfplatte sowie Herstellungsverfahren"" beschrieben ist, ausgebildet ist. Insbesondere ist es bevorzugt, einzelne oder mehrere der darin beschriebenen Ausführungsformen des Einwegbioreaktors mit einzelnen oder mehreren zuvor beschriebenen Ausführungsformen der Anschlussvorrichtung bzw. der Temperiereinrichtung zu kombinieren.

Beschrieben wird ein Verfahren zum Behandeln eines Fluidstromes.

Das Verfahren beinhaltet die Schritte Verbinden einer wiederverwendbaren Temperiereinrichtung mit dem Kopplungselement der Anschlussvorrichtung, Temperieren eines Fluidstromes in einer Fluidleitung mittels der Temperiereinrichtung derart, dass der Fluidstrom wenigstens teilweise kondensiert, und Bereitstellen zumindest eines Teils des Kondensats, welches beim Temperieren des Fluidstromes gebildet wird, für eine Rückführung in einen Einwegbioreaktor. Insbesondere bevorzugt ist eine Fortbildung mit den Schritten: Verbinden einer wiederverwendbaren Temperiereinrichtung mit dem Kopplungselement der Anschlussvorrichtung, Temperieren eines Gasstromes in einer Gasleitung mittels der Temperiereinrichtung derart, dass der Gasstrom wenigstens teilweise kondensiert, und Bereitstellen zumindest eines Teils des Kondensats, welches beim Temperieren des Gasstromes gebildet wird, für eine Rückführung in einen Einwegbioreaktor.

Zu den Vorteilen, Ausführungsvarianten und Ausführungsdetails des Verfahrens und seiner ihrer Fortbildungen wird auf die vorangegangene Beschreibung zu den entsprechenden Vorrichtungsmerkmalen verwiesen.

### Beschreibung bevorzugter Ausführungsbeispiele

Bevorzugte Ausführungsbeispiele werden beispielhaft anhand der beiliegenden Figuren beschrieben. Es zeigen:
- Fig. 1A:: eine dreidimensionale Ansicht einer biotechnologischen Vorrichtung mit einem Einwegbioreaktor, einer Anschlussvorrichtung und einer Temperiereinrichtung;
- Fig. 1B:: eine Seitenansicht der biotechnologischen Vorrichtung gemäß Fig. 1A;
- Fig. 1C:: eine Draufsicht auf die biotechnologische Vorrichtung gemäß Fig. 1A;
- Fig. 1D:: eine Schnittdarstellung der biotechnologischen Vorrichtung gemäß Fig. 1A entlang der Schnittebene A-A in Fig. 1C;
- Fig. 2:: ein paralleles Bioreaktorsystem zur Anwendung der biotechnologischen Vorrichtung;
- Fig. 3:: einen vergrößerten Ausschnitt aus Fig. 1D;
- Fig. 4A, B, C, D:: verschiedene Schritte der Montage einer Anschlussvorrichtung und einer Temperiereinrichtung an einem Abgasschlauch;
- Fig. 5:: eine dreidimensionale Ansicht einer Anschlussvorrichtung und einer Temperiereinrichtung mit zwei Wärmeübertragungselementen; und
- Fig. 6:: eine weitere dreidimensionale Ansicht der Anschlussvorrichtung und Temperiereinrichtung gemäß Fig. 5.

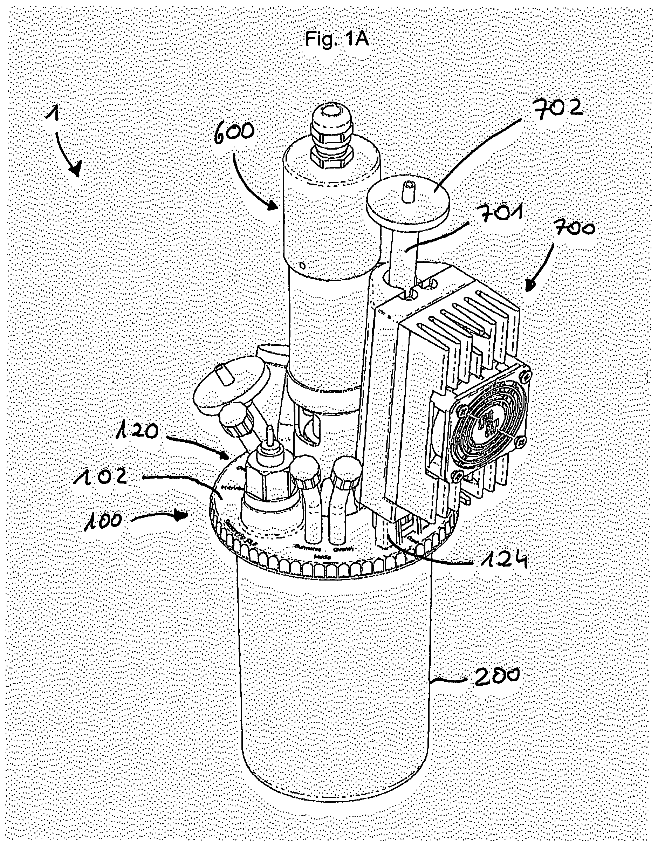

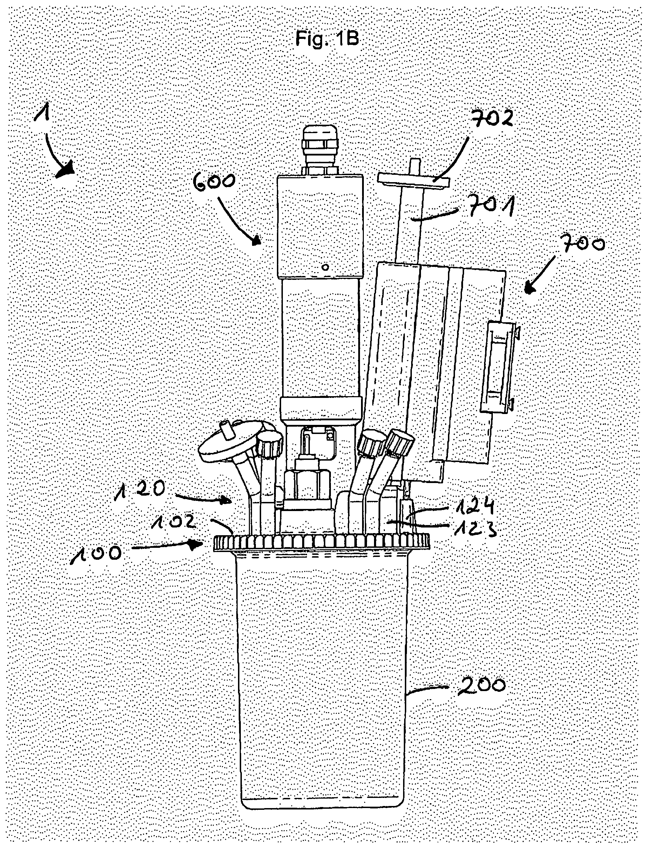

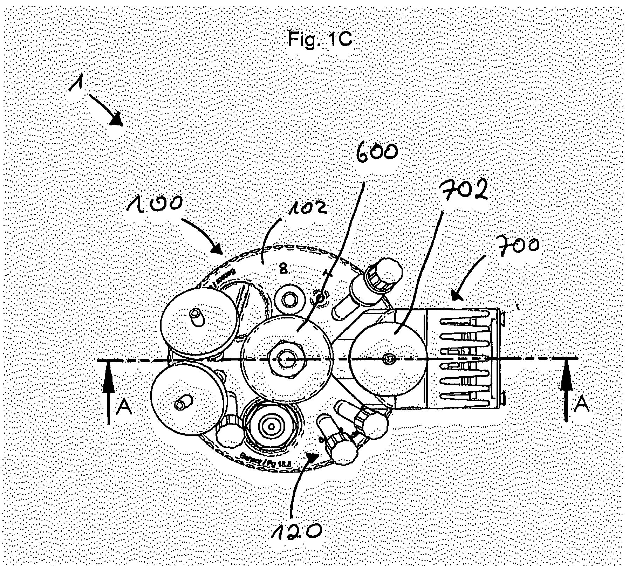

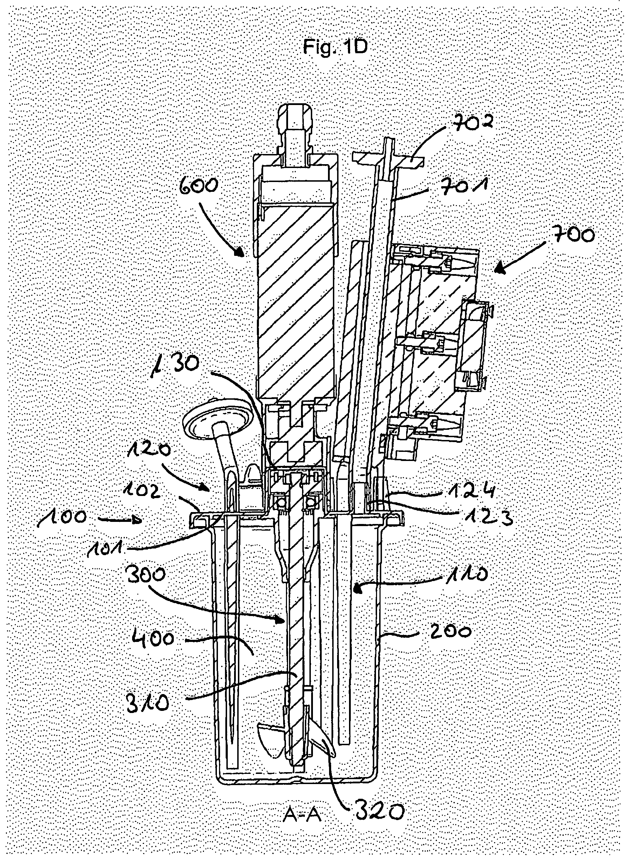

Die Figuren 1 bis 6 stellen beispielhafte Ausführungsbeispiele und deren Anwendung dar. Gleiche oder ähnliche Elemente werden in den Figuren mit den gleichen Bezugszeichen bezeichnet.

Die Fig. 1A,B,C,D zeigen einen Einwegbioreaktor 1 mit einer Kopfplatte 100 und einem Behälter 200, die einen Reaktionsraum 400 einschließen, in dem ein an einer Rührwelle drehsteif befestigtes Rührelement 320 eines Rührwerks angeordnet ist. Die Kopfplatte 100 hat eine dem Reaktionsraum zugewandte Innenseite 101, an der mehrere Tauchrohre 110 angeordnet sind, die in den Reaktionsraum 400 ragen. Auf einer dem Reaktionsraum 400 abgewandten Außenseite 102 der Kopfplatte 100 sind mehrere Anschlüsse 120 angeordnet. Die Kopfplatte 100 ist vorzugsweise einstückig ausgebildet und im Spritzgussverfahren aus vorzugsweise Polyamid hergestellt einschließlich der auf der Außenseite angeordneten Anschlüsse 120 und der auf der Innenseite angeordneten Tauchrohre 110. Die Tauchrohre 110 korrespondieren mit einem Teil der Anschlüsse 120, sodass durch die korrespondierenden Anschlüsse 120 Instrumente, Sensoren, Leitungen, wie z.B. Schläuche, durch die Tauchrohre 110 in den Reaktionsraum ein- bzw. ausgeführt werden können. Die Anschlüsse 120 dienen dazu, die für den Reaktionsprozess notwendigen Stoffe bereitzustellen und/oder Stoffe aus dem Reaktionsraum 400 abzuführen, z.B. beim Betrieb entstehende Gase. Die Anschlüsse 120 können auch als Overlay und die Tauchrohre 110 als Submers bezeichnet werden.

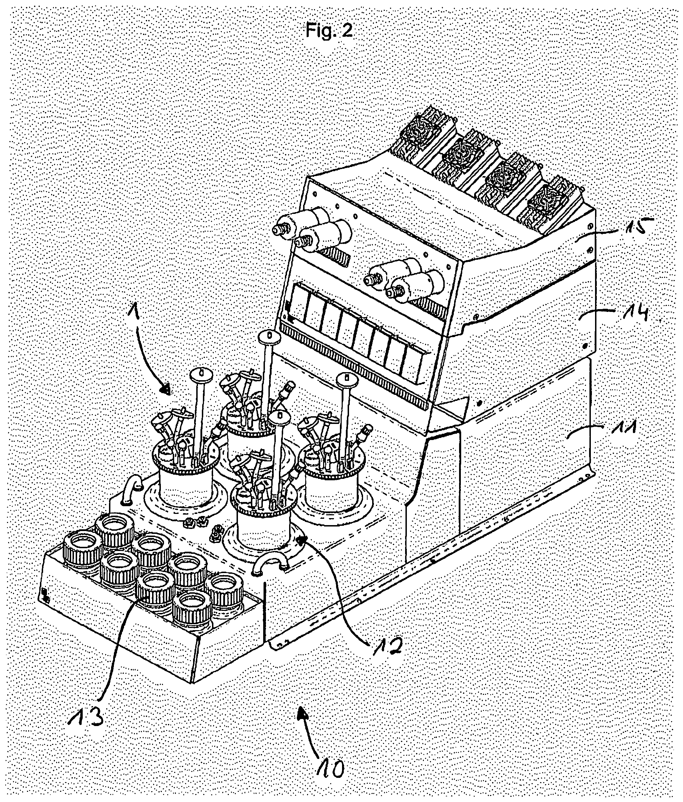

Ein solcher formstabiler Einwegbioreaktor, wie er auch in der parallelen Anmeldung der Anmelderin vom selben Tage mit dem Titel "Einwegbioreaktor und Kopfplatte sowie Herstellungsverfahren" beschrieben ist, ist insbesondere geeignet zur Anwendung in einem parallelen Bioreaktorsystem 10, wie in Fig. 2 gezeigt. Das in Fig. 2 gezeigte parallele Bioreaktorsystem 10 weist einen Basisblock 11 mit vier darin angeordneten Aufnahmen 12 auf, in die jeweils ein Einwegbioreaktor 1 lösbar eingesetzt werden kann. In dem Basisblock 11 ist vorzugsweise eine Temperatursteuerung angeordnet, die ausgebildet ist, die in den Aufnahmen 12 angeordneten Einwegbioreaktoren 1 nach Bedarf zu heizen oder zu kühlen. Angrenzend an den Basisblock 11 ist eine Anordnung mit Behältern 13 ausgebildet. Ferner weist der Basisblock 11 eine Stapelfläche auf, auf der zwei Funktionsblöcke 14, 15 abnehmbar in einer Stapelformation angeordnet sind und beispielsweise als Ablage- und Anzeigestation oder Pumpenstation ausgebildet sind, beispielsweise um die für den Betrieb der Einwegbioreaktoren notwendigen Fluide zu- oder abzuführen. Ein solches paralleles Bioreaktorsystem 10 hat den Vorteil einer hohen Skalierbarkeit, da mehrere dieser parallelen Bioreaktorsysteme 10 mit jeweils vier Einwegbioreaktoren 1 nebeneinander angeordnet werden können.

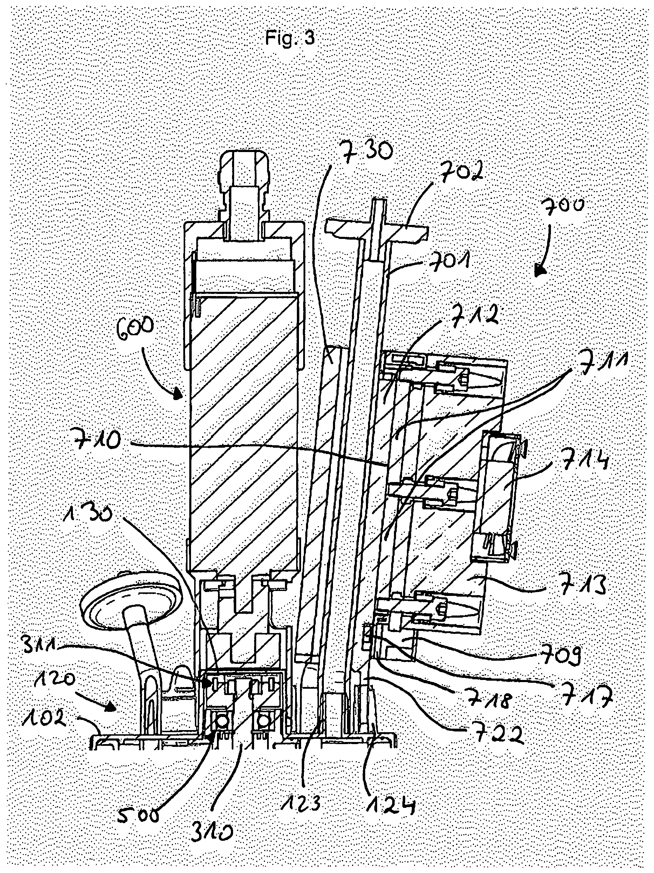

Wie insbesondere in Fig. 3 zu erkennen ist, ist eine Temperiereinrichtung 700 mit einem Zentralelement 709 an einer hier als Abgasschlauch 701 ausgebildeten Gasleitung mit einem Sterilfilter 702 eines Einwegbioreaktors 1 lösbar befestigt. Die folgende Beschreibung zeigt die Anwendung einer Temperiereinrichtung 700 und einer Anschlussvorrichtung 720 für den Anschluss an einen formstabilen Einwegbioreaktor 1 mit einer Kopfplatte 100. Die Temperiereinrichtung 700 und die Anschlussvorrichtung 720 können jedoch ebenso mit einem Einwegbioreaktor mit flexiblen Wandungen verwendet und dort vorzugsweise an ein Anschlusselement angeschlossen werden. Ebenso wird hier die Verwendung der Anschlussvorrichtung und der Temperiereinrichtung zur Temperierung eines Abgasstroms in einem flexiblen Abgasschlauch gezeigt. Ebenso können die Anschlussvorrichtung und die Temperiereinrichtung jedoch auch zur Temperierung von Fluiden in anderen Fluidleitungen und/oder anderen Strömungsrichtungen, beispielsweise einem formstabilen Zu- oder Abgasrohr, verwendet werden.

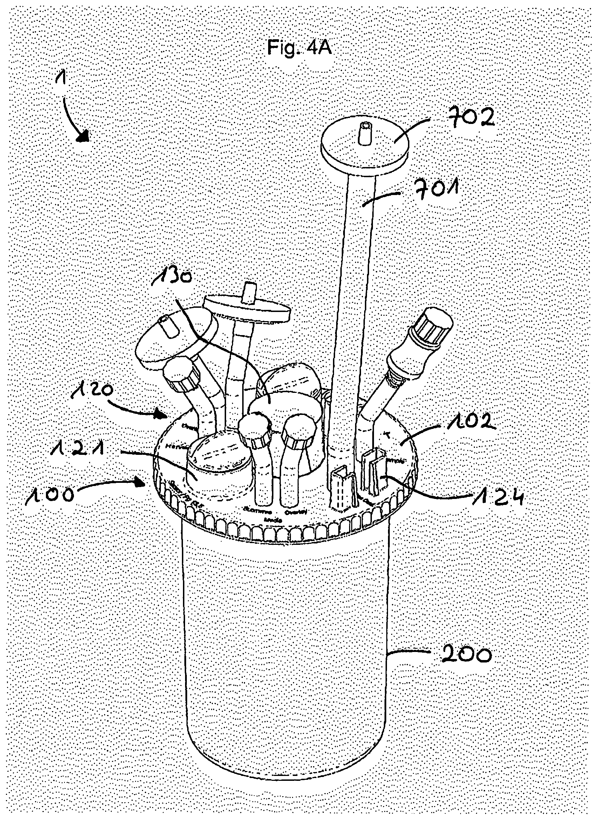

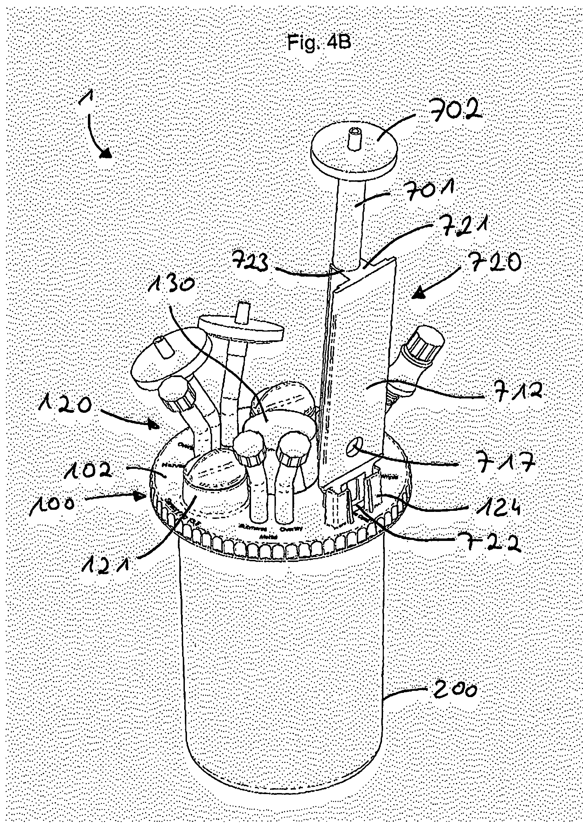

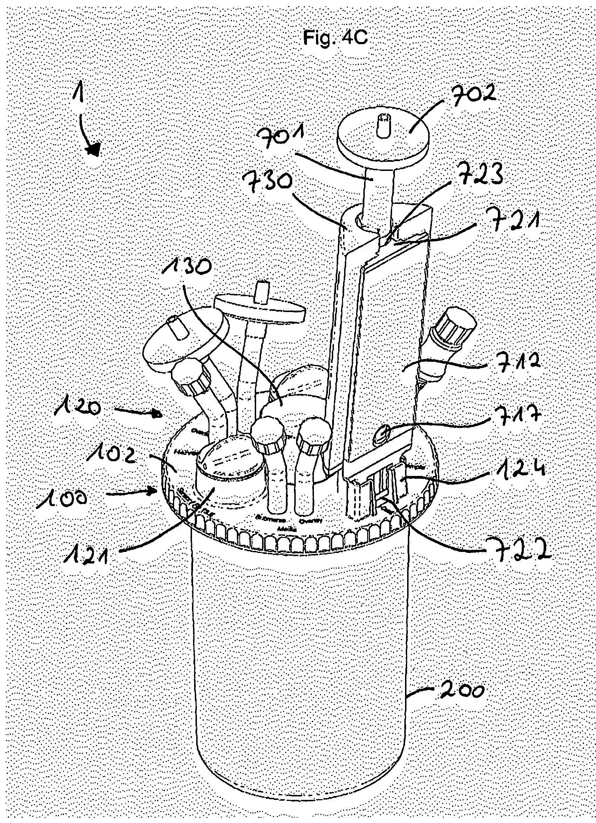

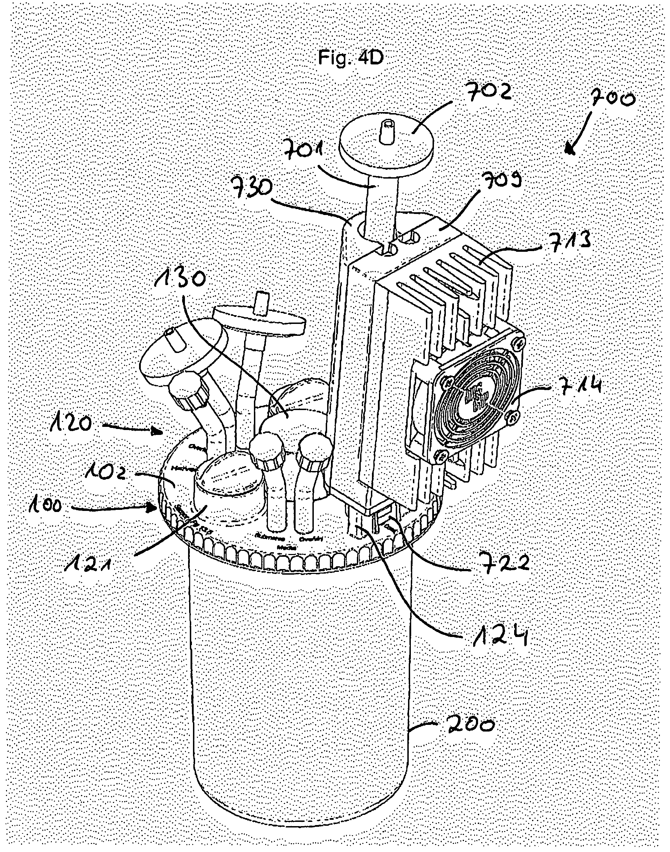

Bei den Darstellungen in den Fig. 4A bis C ist die Temperiereinrichtung 700 von der der Anschlussvorrichtung 720 gelöst, was zur Folge hat, dass die thermische Kopplung zwischen einer Temperierfläche 710 eines von der Temperiereinrichtung 700 umfassten Temperieraktors 711 getrennt ist von dem Kopplungselement 712 der Anschlussvorrichtung 720. Wird die Temperiereinrichtung 700 an der Anschlussvorrichtung 720 montiert, kommen die Temperierfläche 710 und die, hier eben ausgebildete, Koppelfläche des Kopplungselements 712 zur überlappenden Anlage, sodass mit der Montage der Temperiereinrichtung 700 an der Anschlussvorrichtung 720 nicht nur eine mechanische Befestigung, sondern automatisch auch eine thermische Kopplung hergestellt ist.

Der Temperieraktor 711 kann beispielweise mit mindestens einem Peltierelement ausgeführt sein. Die Temperiereinrichtung 700 verfügt in der dargestellten Ausführungsform weiterhin über einen Kühlkörper 713 sowie eine hierauf angeordnete Ventilatoreinrichtung 714.

Die Temperiereinrichtung 700 wird an der Anschlussvorrichtung 720 vorzugsweise mit Hilfe einer mechanischen, selbstarretierten ausgeführten Verbindung befestigt. In den Fig. 3 und 4A bis C ist im unteren Bereich des Kopplungselements 712 ein Sensorikelement 717 zu erkennen, welches zusammen mit einem zugeordneten Sensorikelement 718 (vgl. Fig. 3) an der Temperiereinrichtung 700 Teil einer vorgesehenen Sensorik ist, die die Ankopplung oder Nichtankopplung der Temperiereinrichtung 700 an die Anschlussvorrichtung 720 kontrolliert und im Fall der Entkopplung eine automatische Deaktivierung des Temperieraktors 711 in der Temperiereinrichtung 700 bewirkt. Zum Beispiel handelt es sich bei dem Sensorikelement 717 um einen Licht reflektierenden Spiegel, der ein von dem zugeordneten Sensorikelement 718 ausgesendetes optisches Signal reflektiert, wenn die Temperiereinrichtung 700 angeschlossen ist. In einer anderen Ausführung ist das Sensorikelement 717 mit einem Dauermagneten gebildet, welcher mit einem vom zugeordneten Sensorikelement 718 umfassten Hall-Sensor zusammenwirkt.

Die Schritte zur Herstellung einer Befestigung der Anschlussvorrichtung 720 und der Temperiereinrichtung 700 sind in den Figuren 4A bis D dargestellt. Ein steriler Einwegbioreaktor 1 wird üblicherweise bereits mit einem im Herstellungsprozess ebenfalls sterilisierten und an einem Anschluss 123 der Kopfplatte 100 angebrachten Abgasschlauch 701 mit einem daran angeordneten Sterilfilter 702 bereitgestellt. Die Beibehaltung der Sterilität dieses Systems inklusive des Abgasschlauchs 701 und des Sterilfilters 702 ist von hoher Bedeutung für die Durchführung der biologischen bzw. biotechnologischen Prozesse im Einwegbioreaktor.

Eine Anschlussvorrichtung 720 mit einer als Längsnut ausgebildeten Aufnahme 721 mit einem Kopplungselement 712 sowie einem Verbindungsabschnitt 722 kann an dem Einwegbioreaktor 1 lösbar befestigt werden, ohne dessen Sterilität zu gefährden. Der Verbindungsabschnitt 722 wird in einen dafür vorgesehenen Steckanschluss 124 der Kopfplatte 100 eingesteckt. Ferner wird der flexible Abgasschlauch 701 in einer im Wesentlichen orthogonal zur einer Längsachse der Anschlussvorrichtung 720 verlaufenden Richtung abschnittsweise in die Aufnahme 721 eingedrückt, sodass der Abgasschlauch 701 vorzugsweise klemmend in der Aufnahme 721 sitzt. Eine Kontaktfläche 723 der Aufnahme 721 liegt dabei kontaktierend an einem entsprechenden Flächenabschnitt einer Außenoberfläche des Abgasschlauchs 701 an, um eine möglichst gute Wärmeübertragung zwischen dem Abgasschlauch 701 und der Anschlussvorrichtung 720 herzustellen. Die Längserstreckung der Anschlussvorrichtung 720 ist kürzer als eine Erstreckung des Abgasschlauchs 701 zwischen seiner Verbindung mit dem Einwegbioreaktor 1 und einem am Abgasschlauch 701 angeordneten Sterilfilter 702. Die Längserstreckung der Anschlussvorrichtung 720 macht jedoch einen Großteil der Länge des Abgasschlauchs 701 zwischen seiner Verbindung mit dem Einwegbioreaktor 1 und dem Sterilfilter 702 aus, um eine größere Kontaktfläche und damit eine bessere Wärmeübertragung zu erzielen. Die Anschlussvorrichtung 720 ist vorzugsweise aus Aluminium ausgebildet.

Gemäß Anspruch 1 ist die Anschlussvorrichtung 720 zweiteilig ausgebildet, um den Abgasschlauch 701 möglichst umfänglich, vorzugsweise durch Klemmung und/oder Formschluss, umschließen zu können. Die beiden Teile sind dann ausgebildet, in einer im Wesentlichen orthogonal zur einer Längsachse der Anschlussvorrichtung 720 verlaufenden Richtung zusammengefügt zu werden und dabei den Abschnitt des Abgasschlauchs 701 in der Aufnahme 721 aufzunehmen. Gemäß Anspruch 5 kann die Anschlussvorrichtung zweiteilig ausgebildet sein.

In einem nächsten Schritt wird ein thermisches Isolierelement 730 angeordnet, um den thermischen Wirkungsgrad zu erhöhen und Kondensation an den Außenseiten zu vermeiden. Das thermische Isolierelement 730 kann ebenfalls mit einer Längsnut ausgebildet sein und ebenfalls klemmend und/oder formschlüssig mit dem Abgasschlauch 701 und/oder der Anschlussvorrichtung 720 verbunden sein.

Das thermische Isolierelement 730 ist vorzugsweise aus schlecht wärmeleitenden Materialien, wie beispielsweise geschäumten Polymeren, hergestellt. An das Kopplungselement 712 der Anschlussvorrichtung 720 kann nun thermisch gut koppelnd, vorzugsweise lösbar, eine Temperiereinrichtung 700 angeordnet werden. Die Ausgestaltung der Temperiereinrichtung 700 mit dem vorzugsweise als Peltier-Elementen ausgebildeten Temperieraktor 711, einem Wärmeübertragungs- bzw. Kühlkörper 713 sowie einer darauf angeordneten Ventilatoreinrichtung 714 kann vorzugsweise der in der Anmeldung DE 10 2011 054 364.3 beschriebenen Temperiereinrichtung entsprechen.

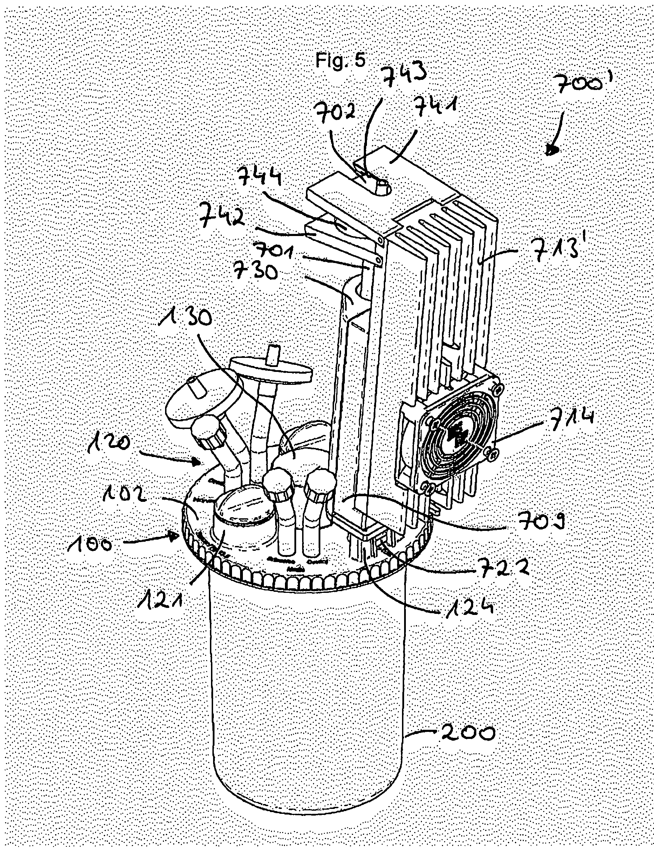

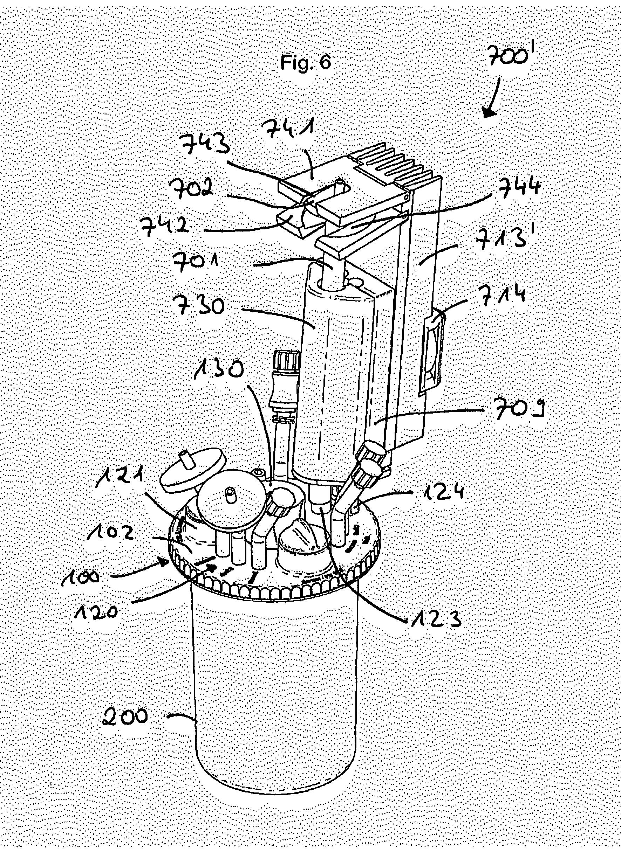

In den Figuren 5 und 6 ist eine Variante der Temperiereinrichtung 700' dargestellt, bei der der Wärmeübertragungskörper 713' verlängert ist, und an diesem um eine horizontale Achse schwenkbar zwei Wärmeübertragungselemente 741, 742 gelagert sind, die plattenförmig mit Schlitzen ausgebildet sind und den Sterilfilter 702 zumindest abschnittsweise umschließen können. Ferner weist das erste Wärmeübertragungselement 741 an seiner dem Sterilfilter 702 zugewandten Seite eine Vertiefung 743 zur Aufnahme eines Teilbereichs des Sterilfilters 702 auf, wobei die Vertiefung 743 vorzugsweise im Wesentlichen als Negativform des aufzunehmenden Teilbereichs des Sterilfilters 702 ausgebildet ist. Ebenso weist das zweite Wärmeübertragungselement 742 an seiner dem Sterilfilter 702 zugewandten Seite eine Vertiefung 744 zur Aufnahme eines Teilbereichs des Sterilfilters 702 auf. Auch die Vertiefung 744 des zweiten Wärmeübertragungselements 742 ist vorzugsweise im Wesentlichen als Negativform des aufzunehmenden Teilbereichs des Sterilfilters 702 ausgebildet.

Dies hat den Vorteil, dass die bei der Abgaskühlung zur Kondensatrückgewinnung entstehende Abwärme nicht einfach nur abgeführt, sondern zur Beheizung des Sterilfilters genutzt wird. Die thermische Kopplung der Wärmeübertragungselemente 741, 742 und des Temperieraktors 711, vorzugsweise der warmen Seite des Temperieraktors, erfolgt vorzugsweise über den Kühlkörper 713'. Die Anordnung von zwei Wärmeübertragungselementen 741, 742 vorzugsweise auf zwei unterschiedlichen Seiten des Sterilfilters 702 führt zu einer verbesserten, gleichmäßigeren Erwärmung des Sterilfilters 702.

## Patentansprüche

1. Wiederverwendbare Temperiereinrichtung (700) zur Temperierung einer sterilen Einweg-Fluidleitung (701) eines Einwegbioreaktors (1),
umfassend einen temperierfluidfreien Temperieraktor (711) und eine Anschlussvorrichtung,
**dadurch gekennzeichnet, dass** die Anschlussvorrichtung eine wiederverwendbare Anschlussvorrichtung (720) zur Verbindung der Temperiereinrichtung (700) mit einer Fluidleitung (701) eines Einwegbioreaktors (1) ist, die Anschlussvorrichtung umfassend
eine Aufnahme (721), in der ein Abschnitt einer Fluidleitung (701), nämlich einer als flexibler Schlauch oder als formstabiles Rohr ausgebildeten Gasleitung, lösbar angeordnet werden kann, und ein Kopplungselement (712) mit einer Koppelfläche zur Verbindung der Anschlussvorrichtung mit einer Temperiereinrichtung (700), wobei die Koppelfläche mit einer Temperierfläche der Temperiereinrichtung in flächigen Kontakt gebracht werden kann, **dadurch gekennzeichnet, dass** die Aufnahme (721) eine Kontaktfläche (723) aufweist, die derart angeordnet und ausgebildet ist, dass die Kontaktfläche (723) an einer in der Aufnahme (721) angeordneten Fluidleitung (701) anliegt, und **dadurch gekennzeichnet, dass** ein Abschnitt einer Fluidleitung (701) in einer im Wesentlichen orthogonal zur einer Längsachse der Anschlussvorrichtung (720) verlaufenden Richtung in die Aufnahme (721) eingeführt werden kann,
**dadurch gekennzeichnet, dass** die Aufnahme (721) einen ersten und einen zweiten Teil aufweist, wobei die beiden Teile ausgebildet sind, in einer im Wesentlichen orthogonal zur einer Längsachse der Anschlussvorrichtung (720) verlaufenden Richtung zusammengefügt zu werden und dabei einen Abschnitt einer Fluidleitung (701) in der Aufnahme (721) aufzunehmen.

2. Temperiereinrichtung (700) nach dem vorhergehenden Anspruch 1,
**gekennzeichnet durch** mindestens ein Wärmeübertragungselement (741) zur Temperierung, insbesondere Erwärmung, eines Sterilfilters (702) eines Einwegbioreaktors (1), wobei das mindestens eine Wärmeübertragungselement (741) mit dem Temperieraktor (711) thermisch koppelnd verbunden ist.

3. Temperiereinrichtung (700) nach dem vorhergehenden Anspruch 2,
**gekennzeichnet durch** ein zweites Wärmeübertragungselement (742) zur Temperierung, insbesondere Erwärmung, eines Sterilfilters (702) eines Einwegbioreaktors (1), wobei das zweite Wärmeübertragungselement (742) mit dem Temperieraktor (711) thermisch koppelnd verbunden ist, wobei vorzugsweise das erste und zweite Wärmeübertragungselement (742) derart angeordnet und ausgebildet sind, dass sie einen Sterilfilter (702) zumindest teilweise umschließen können.

4. Temperiereinrichtung (700) nach einem der beiden vorhergehenden Ansprüche 2-3,
**dadurch gekennzeichnet, dass** das erste und/oder zweite Wärmeübertragungselement (741, 742) plattenförmig, vorzugsweise mit einem Schlitz zur Durchführung einer Gasleitung (701), ausgebildet ist bzw. sind, wobei vorzugsweise das erste und/oder zweite Wärmeübertragungselement (741, 742) auf ihrer jeweiligen einem Sterilfilter (702) zugewandten Seite eine Vertiefung (743, 744) zur Aufnahme eines Teilbereichs des Sterilfilters (702) aufweisen.

5. Biotechnologische Vorrichtung, umfassend
einen Einwegbioreaktor (1) mit einer sterilen Einweg-Fluidleitung (701), nämlich einer als flexibler Schlauch oder als formstabiles Rohr ausgebildeten Gasleitung, und eine wiederverwendbare Temperiereinrichtung (700) zur Temperierung der sterilen Einweg-Fluidleitung (701), umfassend einen temperierfluidfreien Temperieraktor (711) und eine wiederverwendbare Anschlussvorrichtung, wobei die Koppelfläche mit einer Temperierfläche der Temperiereinrichtung in flächigen Kontakt gebracht werden kann, **dadurch gekennzeichnet, dass** die Anschlussvorrichtung eine Anschlussvorrichtung (720) ist zur Verbindung der Temperiereinrichtung (700) mit einer Fluidleitung (701) eines Einwegbioreaktors (1), die Anschlussvorrichtung (720) umfassend eine Aufnahme (721), in der ein Abschnitt einer Fluidleitung (701), lösbar angeordnet werden kann, und ein Kopplungselement (712) mit einer Koppelfläche zur Verbindung der Anschlussvorrichtung mit einer Temperiereinrichtung (700), **dadurch gekennzeichnet, dass** die Aufnahme (721) eine Kontaktfläche (723) aufweist, die derart angeordnet und ausgebildet ist, dass die Kontaktfläche (723) an einer in der Aufnahme (721) angeordneten Fluidleitung (701) anliegt, und **dadurch gekennzeichnet, dass** ein Abschnitt einer Fluidleitung (701) in einer im Wesentlichen orthogonal zur einer Längsachse der Anschlussvorrichtung (720) verlaufenden Richtung in die Aufnahme (721) eingeführt werden kann.

## Claims

1. A reusable temperature control device (700) for controlling the temperature of a sterile single-use fluid conduit (701) of a single-use bioreactor (1), comprising a temperature control actuator (711) which does not contain any temperature control fluid, and a connection device,
**characterized in that** the connection device is a resusable connection device (720) for connecting the temperature control device (700) to a fluid conduit (701) of a single-use bioreactor (1), the connection device comprising
a receptacle (721) in which a portion of a fluid conduit (701), namely a gas conduit embodied as a flexible tube or as a dimensionally stable pipe, can be detachable arranged, and a coupling member (712) having a coupling face for connecting the connection device to a temperature control device (700), wherein the coupling face can be brought into planar contact with a temperature control surface of the temperature control device, **characterized in that** the receptacle (721) has a contact surface (723) which is arranged and adapted such that the contact surface (723) abuts a fluid conduit (701) arranged in the receptacle (721), and **characterized in that** a portion of a fluid conduit (701) can be introduced into the receptacle (721) in a direction running substantially orthogonal to a longitudinal axis of the connection device (720),
**characterized in that** the receptacle (721) has a first and a second part, the two parts being adapted to be joined together in a direction running substantially orthogonally to a longitudinal axis of the connection device (720) and to receive a portion of a fluid conduit (701) in the receptacle (721) thereby.

2. The temperature control device (700) according to claim 1,
**characterized by** at least one heat transmission member (741) for controlling the temperature of, in particular for heating, a sterile filter (702) of a single-use bioreactor (1), wherein the at least one heat transmission member (741) is connected thermocouplingly to the temperature control actuator (711).

3. The temperature control device (700) according to claim 2,
**characterized by** a second heat transmission member (742) for controlling the temperature of, in particular for heating, a sterile filter (702) of a single-use bioreactor (1), the second heat transmission member (742) being connected thermocouplingly to the temperature control actuator (711), the first and second heat transmission member (742) preferably being adapted and arranged such that they can at least partially enclose a sterile filter (702).

4. The temperature control device (700) according to any one of the two preceding claims 2-3,
**characterized in that** that the first and/or second heat transmission members (741, 742) are embodied in plate-shaped form, preferably with a slot for a gas conduit (701) to pass through, wherein preferably the first and/or second heat transmission member (741, 742) comprise on their respective sides facing the sterile filter (702) an indentation (743, 744) for receiving at least a part of the sterile filter (702).

5. A biotechnological device comprising
a single-use bioreactor (1) provided with a sterile single-use fluid conduit (701), namely a gas conduit embodied as a flexible tube or as a dimensionally stable pipe, and a reusable temperature control device (700) for controlling the temperature of the sterile single-use fluid conduit (701), comprising a temperature control actuator (711) which does not contain any temperature control fluid, and a reusable connection device, wherein the coupling face can be brought into planar contact with a temperature control surface of the temperature control device, **characterized in that** the connection device is a connection device (720) for connecting the temperature control device (700) to a fluid conduit (701) of a single-use bioreactor (1), the connection device comprising a receptacle (721) in which a portion of a fluid conduit (701) can be detachable arranged, and a coupling member (712) having a coupling face for connecting the connection device to a temperature control device (700), **characterized in that** the receptacle (721) has a contact surface (723) which is arranged and adapted such that the contact surface (723) abuts a fluid conduit (701) arranged in the receptacle (721), and **characterized in that** a portion of a fluid conduit (701) can be introduced into the receptacle (721) in a direction running substantially orthogonal to a longitudinal axis of the connection device (720).

## Revendications

1. Système de thermorégulation (700) réutilisable servant à thermoréguler une conduite de fluide stérile à usage unique (701) d'un bioréacteur à usage unique (1),
comprenant un réacteur de thermorégulation (711) exempt de fluide de thermorégulation et un dispositif de raccordement,
**caractérisé en ce que** le dispositif de raccordement est un dispositif de raccordement (720) réutilisable servant à relier le système de thermorégulation (700) à une conduite de fluide (701) d'un bioréacteur à usage unique (1), le dispositif de raccordement comprenant
un logement (721), dans lequel un tronçon d'une conduite de fluide (701), à notamment une conduite de gaz constituée d'un tube flexible ou d'un conduit indéformable, peut être disposé de manière amovible, et un élément de couplage (712) pourvu d'une surface de couplage servant à relier le dispositif de raccordement à un système de thermorégulation (700), la surface de couplage pouvant être mise en contact plan avec une surface de régulation de température du système de thermorégulation,
**caractérisé en ce que** le logement (721) présente une surface de contact (723), qui est disposée et réalisée de telle manière que la surface de contact (723) repose au niveau d'une conduite de fluide (701) disposée dans le logement (721), et **caractérisé en ce qu'**un tronçon d'une conduite de fluide (701) peut être introduit dans le logement (721) dans une direction s'étendant essentiellement de manière orthogonale par rapport à un axe longitudinal du dispositif de raccordement (720),
**caractérisé en ce que** le logement (721) présente une première partie et une deuxième partie, sachant que les deux parties sont réalisées pour être assemblées dans une direction s'étendant essentiellement de manière orthogonale par rapport à un axe longitudinal du dispositif de raccordement (720) et, pour ce, faisant loger un tronçon d'une conduite de fluide (701) dans le logement (721).

2. Système de thermorégulation (700) selon la revendication précédente 1,
**caractérisé par** au moins un élément de transmission de chaleur (741) servant à thermoréguler, en particulier à réchauffer, un filtre stérile (702) d'un bioréacteur à usage unique (1), sachant que l'élément de transmission de chaleur (741), au moins au nombre de un, est relié par un couplage thermique au réacteur de thermorégulation (711).

3. Système de thermorégulation (700) selon la revendication précédente 2,
**caractérisé par** un deuxième élément de transmission de chaleur (742) servant à thermoréguler, en particulier à réchauffer, un filtre stérile (702) d'un bioréacteur à usage unique (1), sachant que le deuxième élément de transmission de chaleur (742) est relié par un couplage thermique au réacteur de thermorégulation (711), sachant que de préférence les premier et deuxième éléments de transmission de chaleur (742) sont disposés et réalisés de telle manière qu'ils peuvent renfermer au moins en partie un filtre stérile (702).

4. Système de thermorégulation (700) selon l'une quelconque des deux revendications précédentes 2 à 3,
**caractérisé en ce que** le premier et/ou le deuxième élément de transmission de chaleur (741, 742) sont réalisés de manière à présenter une forme de plaque, de préférence avec une entaille pour le passage d'une conduite de gaz (701), sachant que de préférence le premier et/ou le deuxième élément de transmission de chaleur (741, 742) présentent, sur leur côté respectif tourné vers un filtre stérile (702), un renfoncement (743, 744) servant à loger une zone partielle d'un filtre stérile (702).

5. Dispositif biotechnologique, comprenant
un bioréacteur à usage unique (1) pourvu d'une conduite de fluide stérile à usage unique (701), à notamment une conduite de gaz constituée d'un tube flexible ou d'un conduit indéformable, et un système de thermorégulation (700) réutilisable servant à thermoréguler la conduite de fluide stérile à usage unique (701), comprenant un réacteur de thermorégulation (711) exempt de fluide de thermorégulation et un dispositif de raccordement réutilisable, la surface de couplage pouvant être mise en contact plan avec une surface de régulation de température du système de thermorégulation, **caractérisé en ce que** le dispositif de raccordement est un dispositif de raccordement (720) servant à relier le système de thermorégulation (700) à une conduite de fluide (701) d'un bioréacteur à usage unique (1), le dispositif de raccordement (720) comprenant
un logement (721), dans lequel un tronçon d'une conduite de fluide (701) peut être disposé de manière amovible, et un élément de couplage (712) pourvu d'une surface de couplage servant à relier le dispositif de raccordement à un système de thermorégulation (700),
**caractérisé en ce que** le logement (721) présente une surface de contact (723), qui est disposée et réalisée de telle manière que la surface de contact (723) repose au niveau d'une conduite de fluide (701) disposée dans le logement (721), et **caractérisé en ce qu'**un tronçon d'une conduite de fluide (701) peut être introduit dans le logement (721) dans une direction s'étendant essentiellement de manière orthogonale par rapport à un axe longitudinal du dispositif de raccordement (720).
